# EUROPEAN PATENT SPECIFICATION

(11) **EP 1 981 860 B1**
(45) Date of publication and mention of the grant of the patent: **25.05.2011**
(21) Application number: 07706182.8
(22) Date of filing: 12.01.2007
(51) Int. Cl.: C07D 413/06, C07D 249/08, C07D 263/20, C07C 241/02

(54) **CONVERSION OF AROMATIC DIAZONIUM SALT TO ARYL HYDRAZINE**
UMWANDLUNG EINES AROMATISCHEN DIAZONIUMSALZES IN EIN ARYLHYDRAZIN
CONVERSION D'UN SEL DE DIAZONIUM AROMATIQUE EN ARYLHYDRAZINE

(30) Priority: 19.01.2006 IN CH00902006
(43) Date of publication of application: 22.10.2008
(73) Proprietor: Matrix Laboratories Ltd, Secunderabad 500 003, Andhra Pradesh (IN)
(72) Inventor: RAY, Purna, Chandra, Andhra Pradesh (IN); MEDIKONDURI, Sreekanth, Secunderabad - 500 003 (IN); RAMANJANEYULU, Gorantla, Seeta, Secunderabad 500 003 (IN)
(74) Representative: Wittkopp, Alexander
(86) International application number: PCT/IN2007/000011
(87) International publication number: WO 2007/083320

(56) References cited:
- WO-A1-95/32197
- WO-A1-97/06162
- WO-A1-2004/014901
- US-A- 5 399 574
- HORNER L. ET AL.: "Phosphororganische Verbindungen, XXVI. Darstellung und Eigenschaften einiger p-Chinontriphenyl-phosphazine" CHEMISCHE BERICHTE, vol. 94, no. 5, 1961, pages 1326-1330, XP002572813 DOI: 10.1002/cber.19610940524
- 'Dae-Dong Sung' BULL KOREAN CHEM. SOC. vol. 18, no. 9, 1997, pages 945 - 951, XP008128994

## Description

The present invention relates to the conversion of aromatic diazonium salt into aryl hydrazine. The aryl hydrazine thus formed can be pharmacologically useful compounds in treating a variety of ailments

### Background of the invention:

Class of 5-substituted tryptamines with pharmacological activity at 5-HT 1D and other monoamine receptors such as Zolmitriptan, Rizatriptan, Sumatriptan, Almotriptan, Naratriptan and Eletriptan are known for their therapeutic targets for the treatment of migraine.

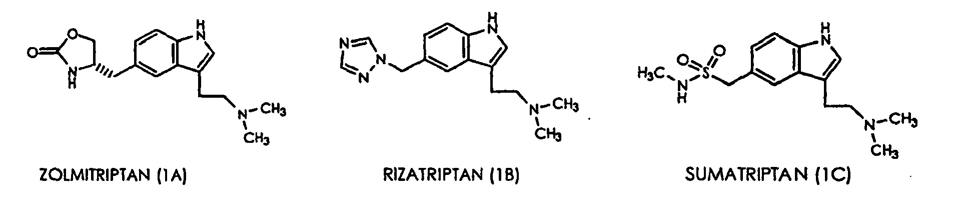

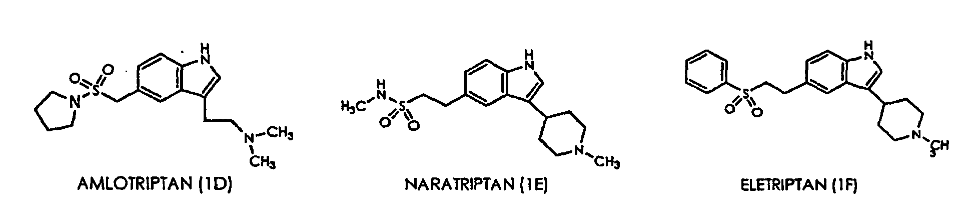

A common structural feature in many anti-migraine compounds is the presence of a 5-substituted indole moiety. Compounds such as Rizatriptan, Zolmitriptan, Sumatriptan, Almotriptan, Naratriptan, Eletriptan and the like, have a moiety present in their structure.

Fisher indole reaction of aryl hydrazine derivatives is well known reaction in literature. Many of the literature processes involves either isolation of that aryl hydrazine derivatives as a salt or taken directly in-situ for Fisher cyclization to give 5-substituted tryptamines analogues. The reduction of diazonium salt to hydrazine derivative in highly acidic medium by sodium sulphite or tin chloride is well known.

U.S. Pat. No.5,399,574 describes a method for preparing Zolmitriptan, and related compounds wherein the conversion of diazonium salt to hydrazine derivative is carried out in presence of tin chloride in concentrated hydrochloric acid followed by Fisher Indole cyclisation to give the desired 5-substituted tryptan. U.S. Pat. No. 5,298,520 discloses similar procedure for the synthesis of different 5-substituted tryptamines wherein the use of tin chloride is reported in presence of aqueous hydrochloric acid for the conversion of aromatic diazonium salt to hydrazine derivatives. U.S. Pat. No's. 5,567,819 and 6,084,103 also describes a process for the preparation of the Rizatriptan and related molecules wherein the conversion of aryl hydrozonium salt to hydrazine is carried out with sodium sulphite in highly acidic medium. All the above processes require tin chloride as a reducing agent which is not easy to remove from the reaction mixture and is required more equivalents for reaction completion. The above processes make the finished product expensive. Isolation of the product from such reaction mixture is tedious, requires several critical layer separations, multi step column chromatography which ultimately gives low yield of the product.

Surprisingly the present inventors have found that by using two moles of triphenyl phosphine for the conversion of diazonium salt to hydrazine derivative is a superior alternative to the existing tin chloride or sodium sulphite process.

### Summary of the invention:

In one aspect the present invention is provided a process for the conversion of an aryl diazonium salt of formula-II wherein R is selected from A,B,C,D,F and to aryl hydrazine of formula - III, 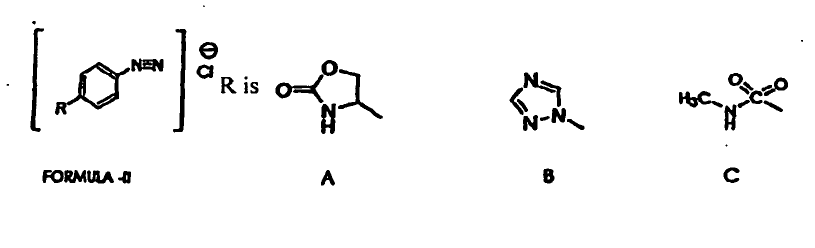 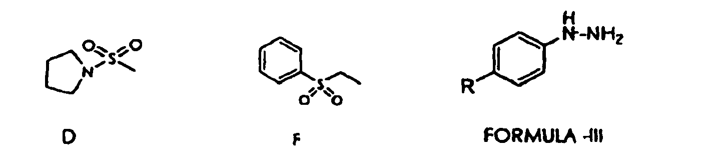 with triphenyl phosphine in presence of an organic solvents.

In another aspect is provided a process for the preparation Zolmitriptan, or substituted derivative of formula -IA or salt thereof 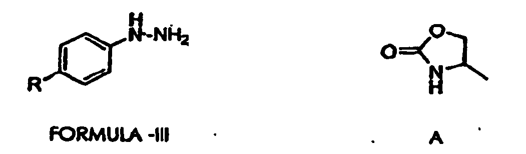 wherein R is a designated residual of formula-A, which comprises reacting in-situ intermediate of formula - II with triphenyl phosphine in methanol followed by fisher indole cyclization with N,N,dimethyl amino acetal

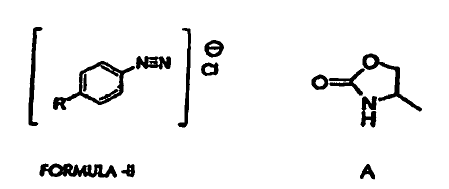

### Detailed description of the invention:

In accordance with the present invention aryl hydrazines of Formula-III wherein R is defined as above are being prepared by
- Converting the aryl amine derivates to their diazonium salt
- Treating the diazonium salt with Triphenyl phosphine to get triphenyl-aryl hydrazyl phosphonium salt
- Hydrolysis of triphenyl-aryl hydrazyl phosphonium salt to yield aryl hydrazine or its salt thereof.

The reaction scheme can be expressed as follows

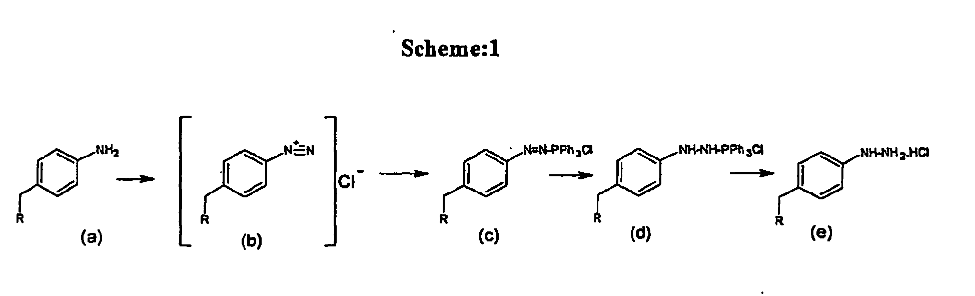

In a specific embodiment, the present invention provides a process for the preparation of aryl hydrazines, which involves
- Dissolving the respective aryl amine in a mineral acid selected from Hydrochloric acid, Hydrobromic acid and Sulphuric acid
- Cooling the solution to -15 to -10°C
- Adding the above cooled solution to a solution of sodium nitrite in a molar ratio of 1.0 to 3.0 moles preferably 1.10 to 1.20 moles dissolved in water
- Maintaining the reaction mass at -15 to -5°C, preferably at about -10°C for the reaction completion to get aryl diazonium salt.

Further the obtained aryl diazonium salt is converted to aryl hydrazine derivative by,
- Adding the diazonium salt to a solution of triphenyl phosphine in a molar ratio of 2.0 to 5.0 moles preferably 3.0 moles in methanol at about 5 to 10°C.
- Adding water after complete addition of triphenyl phosphine
- Stirring the reaction mass at room temperature for about 6 to 8 hrs.
- Adding methanol, Conc.HCl and water one after another to the above reaction mass.
- Heating the resulting solution to reflux temperature and maintaining for 4 hrs.
- Cooling the reaction mass and washing with water immiscible organic solvent selected from methylene chloride, chloroform, toluene, hexane, ethyl acetate etc. to remove the impurities.
- Distilling the reaction mass to remove methanol and water under vacuum to get residue
- Triturating the residue with an organic solvent to get the desired salt of aryl hydrazine derivative

However, where the intermediate aryl hydrazine salt is not isolated the crude is proceeded directly for the Fisher Indole cyclization reaction.

Triphenyl phosphine reacts with diazonium salt results a red color intermediate in a very good yield of triphenyl-aryl hydrazyl phosphonium salt, which practically disintegrate with the acidic hydrolysis quantitatively yields aryl hydrazine. Triphenyl phosphine is easy to use in commercial scale and this method is equivalent to the known method for representation of aryl hydrazine from aryl diazonium salt.

The hydrolysis of phosphonium salt with hydrochloric acid leads quantitative yield to the corresponding aryl hydrazine, which can be isolated as salt or taken in-situ to the formation of triptamine by fisher indole cyclization.

The invention is further illustrated with a few non-limiting examples

### General procedure for the preparation of aryl hydrazine hydrochlore:-

A solution of respective aryl amine (1.0 mole) in conc. HCl (4.0 vol) is cooled to -15 to - 10°C and added slowly to a solution of sodium nitrite (1.10 moles) in water (4 Times). Reaction mass is maintained at -10°C for about 1 hr. for the completion of reaction to get the aryl diazonium chloride.

The obtained aryl diazonium chloride is added to a solution of triphenyl phosphine (3.0 moles) in methanol (2.0 vol) at 5 to 10°C followed by water (6.0 vol) is added and maintained at room temperature for about 6 to 8 hrs. Finally to the above solution methanol (6.0 vol) Conc. HCl (6.0 vol) and water (6.0 vol) are added one after another. Heated the resulting solution to reflux temperature for 4 hrs. Cooled the reaction mass and washed with methylene chloride to remove the impurities. Distilled off methanol and water mixture under vacuum and the residue is triturated with Isopropyl alcohol to get the desired hydrazine hydrochloride with 90% yield.

However, where the intermediate hydrazine hydrochloride is not isolated is directly taken as such for the Fisher Indole cyclization reaction.

For example aryl hydrazine hydrochloride in Almotriptan in highly unstable and the colour immediately turns from pale yellow to dark brown. Hence, the reaction will be proceeded insitu to further step.

### Example-1: Preparation of (S)-4-(4-methylphenyl hydrazine)-1,3-oxazolidin-2-one

(S)-4-4-(amino benzyl)-1,3- oxazolidine-2-one (10 g, 0.052 moles) is suspended in water (20 ml) and cooled to -5°C. A solution of sodium Nitrite (4.2 g; 0.06 moles in 42.0 ml water) is slowly added to the above solution and stirred for 45 min. at -5 to 0°C.

The above reaction mixture is added lot-wise at 5°C to 10°C to a solution of Triphenyl phosphine (40.91 g; 0.155 moles) in methanol (120.0 ml) and diethyl ether (40.0 ml).The reaction mixture is stirred at room temperature for about 8hrs. After the reaction is completed the resulted dark brown oily layer is separated and evaporated under vacuum. The obtained residue is triturated with chloroform and methanol to get the desired product (35.0g).

### Example - 2: Preparation of 1-(4-hydrazinophenyl) methyl-1, 2,4-traizole.

4-[1H-1,2,4-trizole-1-yl methyl] benzene amine (5.0 g;0.028 moles) is suspended in con. Hydrochloric acid (20 ml) and cooled to -15 to -10°C. A solution of sodium Nitrite (2.2 g; 0.030 moles in water 8.96 ml) is slowly added to the above solution and stirred for 60 min at about-10°C.

The above reaction mixture is added lot-wise at 5°C to 10°C to a solution of Triphenyl phosphine (22.67 g; 0.086 moles) in methanol (44 ml) and diethyl ether (110.0 ml).The reaction mixture is stirred at room temperature for about 8 hrs. After the reaction is completed methanol (30 ml) Conc. Hydrochloric acid (30 ml) and water (30 ml) are added to the reaction mixture and maintained the reaction mixture at reflux temperature for 5 hrs. Cooled the reaction mass to room temperature and washed the reaction mass with methylene chloride (5 x 50 ml) and distilled out methanol and water completely in vacuum. The residue is triturated with IPA (50 ml) to give the desired product as off-white solid (6.3 g).

### Example-3: Preparation of 4(-pyrrolidono sulfonyl methyl) phenyl hydrazine

4-(Pyrrolidino sulfonyl methyl) benzene amine (50.0 g; 0.022 moles) is suspended in con. Hydrochloric acid (30 ml) and cooled to -15 to -10°C. A solution of sodium Nitrite (1.97 g; 0.028 moles in water 19.85 ml) is slowly added to the above solution over 30 min and stirred for 60 min at about -10°C.

The above reaction mixture is added lot-wise at 5°C to 10°C to a solution of Triphenyl phosphine (17.31 g; 0.066 moles) in methanol (34.6 ml) and diethyl ether (104 ml) over 2 hrs. To the reaction mass DM water (30 ml) is added and maintained for 8 hrs at room temperature. After the reaction is completed methanol (30 ml) Conc. Hydrochloric acid (30 ml) and water (30 ml) are added to the reaction mixture and maintained the reaction mixture at reflux temperature for 5 hrs. Cooled the reaction mass to room temperature and washed the reaction mass with methylene chloride (5 x 50 ml) and distilled out methanol and water completely in vacuum. The residue is triturated with IPA (50 ml) to give the desired product as off-white solid (3.5 g).

## Claims

1. A process for the preparation of aryl hydrazine of Formula 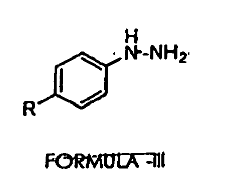 or its salt,
Wherein 'R' is selected from A, B, C, D,F and 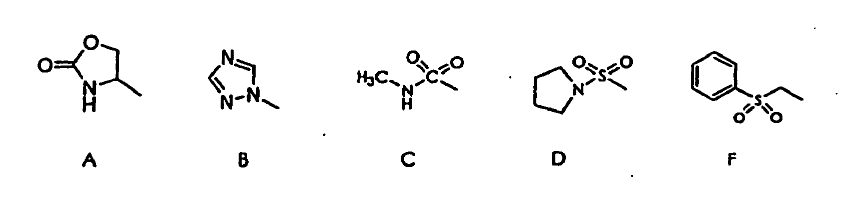 wich comprises
• Dissolving the respective aryl amine in a mineral acid
• Adding the above cooled solution to a solution of sodium nitrite dissolved in water
• Maintaining the reaction mass to get aryl diazonium salt
• Adding the diazonium salt to a solution of triphenyl phosphine
• Adding water after addition of triphenyl phosphine and stirring the reaction mass for the formation of triphenyl aryl hydrazyl-phosphomium salt
• Hydrolysis of triphenyl aryl hydrazyl-phosphomium salt
• optionally washing the reaction mass with water immiscible organic solvent(s) to remove impurities
• optionally isolating the aryl hydrazine as desired salt

2. The process as claimed in claim 1, wherein the mineral acid is selected from Hydrochloric acid, Hydrobromic acid and Sulphuric acid

3. The process as claimed in claim 1, wherein in the sodium nitrite is used in a molar ratio of 1.0 to 3.0 moles preferably 1.10 to 1.20 moles

4. The process as claimed in claim 1, wherein in triphenyl phosphine is used in a molar ratio of 2.0 to 5.0 moles preferably 3.0 moles

5. The process as claimed in claim 1, wherein in the water immiscible solvent is selected from methylene chloride, chloroform, toluene, hexane, ethyl acetate

6. The process as claimed in claim 1, wherein the isolation of aryl hydrazine desired salt optionally involves concentration and trituration with organic solvent(s)

7. The process as claimed in claim 1, wherein in the solvent used for trituration is selected from methanol, isopropanol, chloroform, methylene chloride and mixtures thereof

8. The process as claimed in claim 1, wherein the aryl hydrazine of the formula - III is isolated or with out isolation proceeded for further steps

## Patentansprüche

1. Verfahren für die Herstellung eines Aryl Hydrazins der Formel 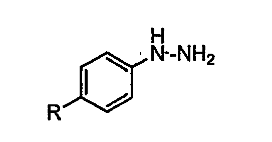 oder eines Salzes davon, wobei:
"R" ausgewählt ist, aus A, B, C, D und F 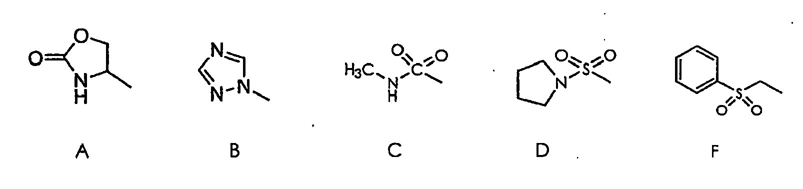 umfassend
• lösen des entsprechenden Arylamins in einer Mineralsäure
• zugeben der obigen, gekühlten Lösung zu einer Lösung von Natriumnitrit in Wasser
• erhalten des Reaktionsgemischs, um ein Aryldiazoniumsalz zu erhalten
• Zugabe des Diazoniumsalzes zu einer Lösung von Triphenylphosphin
• Zugabe von Wasser nach Zugabe von Triphenylphosphine und Rühren des Reaktionsgemisches bis zur Bildung des Triphenylarylhydrazylphosphoniumsalzes
• Hydrolyse des Triphenylarylhydrazylphosphoniumsalzes
• ggf. Waschen des Reaktionsgemischs mit einem mit Wasser nicht mischbaren organischen Lösungsmittel, um Verunreinigungen zu entfernen
• ggf. Isolierung des Arylhydrazines in Form des erwünschten Salzes

2. Verfahren nach Anspruch 1, wobei
die Mineralsäure aus Salzsäure, Bromwasserstoffsäure und Schwefelsäure ausgewählt ist.

3. Verfahren nach Anspruch 1, wobei
das Natriumnitrit in einem molaren Verhältnis von 1,0-3,0 mol, bevorzugt 1,1-1,2 mol, verwendet wird.

4. Verfahren nach Anspruch 1, wobei
das Triphenylphosphin in einem molaren Verhältnis von 2,0-5,0 mol, bevorzugt 3,0 mol, verwendet wird.

5. Verfahren nach Anspruch 1, wobei
das mit Wasser nicht mischbare Lösungsmittel ausgewählt ist aus Methylenchlorid, Chloroform, Toluol, Hexan du Ethylacetat.

6. Verfahren nach Anspruch 1, wobei
die Isolierung des erwünschtes Arylhydrazinsalzes optional die Konzentration und Behandlung mit einem organischen Lösungsmittel umfasst.

7. Verfahren nach Anspruch 1, wobei
das Lösungsmittel zur Behandlung ausgewählt ist aus Methanol, Isopropanol, Chloroform, Methylenchlorid sowie Mischungen davon.

8. Verfahren nach Anspruch 1, wobei
das Arylhydrazin der Formel III isoliert oder nicht isoliert weiteren Verfahrensschritten unterzogen wird.

## Revendications

1. Procédé pour la préparation d'arylhydrazine de formule 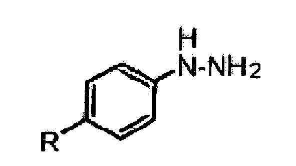 ou d'un sel de celle-ci,
'R' étant sélectionné parmi A, B, C, D et F, 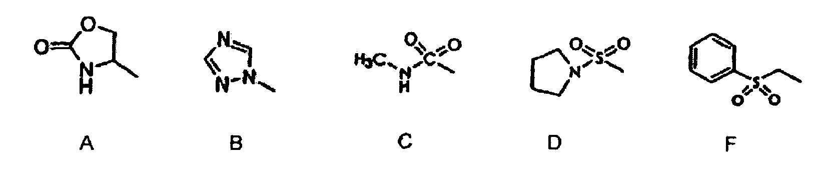 qui comprend les étapes consistant à :
• dissoudre l'arylamine respective dans un acide minéral
• ajouter la solution refroidie ci-dessus à une solution de nitrite de sodium dissoute dans de l'eau
• maintenir la masse réactionnelle pour obtenir un sel d'aryle diazonium
• ajouter le sel de diazonium à une solution de triphényle phosphine
• ajouter de l'eau après addition de triphényle phosphine et agiter la masse réactionnelle pour la formation du sel de triphényl-aryl hydrazyle phosphonium
• hydrolyser le sel de triphényl-aryl hydrazyle phosphonium
• de manière optionnelle, laver la masse réactionnelle avec un ou des solvants organiques non miscibles dans l'eau pour retirer les impuretés.
• de manière optionnelle, isoler l'arylhydrazyl sous la forme du sel souhaité.

2. Procédé selon la revendication 1, dans lequel l'acide minéral est sélectionné parmi acide hydrochrolorique, acide hydrobromique et acide sulfurique.

3. Procédé selon la revendication 1, dans lequel le nitrite de sodium est utilisé en un rapport molaire de 1,0 à 3,0 moles, de manière préférée 1,10 à 1,20 moles.

4. Procédé selon la revendication 1, dans lequel la triphényle phosphine est utilisée en un rapport molaire de 2,0 à 5,0 moles, de manière préférée 3,0 moles.

5. Procédé selon la revendication 1, dans lequel le solvant non miscible dans l'eau est sélectionné parmi chlorure de méthylène, chloroforme, toluène, hexane, éthylacétate.

6. Procédé selon la revendication 1, dans lequel l'isolement du sel souhaité d'arylhydrazine implique de manière optionnelle une concentration et une trituration avec un ou des solvants organiques.

7. Procédé selon la revendication 1, dans lequel le solvant utilisé pour la trituration est sélectionné parmi méthanol, isopropanol, chloroforme, chlorure de méthylène et des mélanges de ceux-ci.

8. Procédé selon la revendication 1, dans lequel l'arylhydrazine selon la formule est isolée ou est soumis aux étapes ultérieures sans isolation.
